# EUROPÄISCHE PATENTANMELDUNG

(11) **EP 3 760 325 A1**
(43) Veröffentlichungstag der Anmeldung: **06.01.2021**
(21) Anmeldenummer: 19405010.0
(22) Anmeldetag: 04.07.2019
(51) Int. Cl.: B08B 9/42, A61L 2/20, B08B 7/00, B67C 7/00

(54) **PRODUKTIONSLINIE ZUR DEKONTAMINATION VON FLIESSBANDARTIG DURCH EINE DEKONTAMINATIONSEINHEIT GELEITETEN STÜCKGUTARTIKELN**

(71) Anmelder: SKAN AG, 4123 Allschwil (CH)
(72) Erfinder: Hommes, Gregor, 47669 Wachtendonk (DE)
(74) Vertreter: Ullrich, Gerhard

(57) **Zusammenfassung**

Produktionslinie (**1**) mit Dekontaminationseiheit zur Dekontamination von Behältnissen für pharmakologische, biotechnische, chemische oder lebensmittel-technische Substanzen oder Stopfen oder Verschlusskappen, insbesondere aus Gummi oder Kunststoff, zum Verschliessen solcher Behältnisse. Zum Transfer der Stückgutartikel (**9**) durch die Dekontaminationseinheit (**3**) sind mehrere miteinander in Reihe verkettete Förderer (**25.1-25.4**) vorgesehen, die Andockstellen (**26a-26n**) besitzen, zu denen jeweils ein Pneumatikkanal (**27**) führt. An den Mündungen der Andockstellen (**26a-26n**) herrscht ein die Stückgutartikel (**9**) haltender Sog. Am jeweils nächsten Förderer (**25.2-25.4**) wird eine andere Artikelfläche (**92,93**) angesaugt, als am vorherigen Förderer (**25.1-25.3**), so dass schliesslich die gesamte Oberfläche jedes Stückgutartikels (**9**) der Wirkung einer in der Dekontaminationseinheit vorhandenen Dekontaminationsvorrichtung (**30**) ausgesetzt ist, welche als Strahlungsquelle, z.B. Elektronenstrahler oder als Begasungseinrichtung zur Vernebelung einer Biozid-Lösung ausgebildet ist.

## Beschreibung

### Anwendungsgebiet der Erfindung

Die Erfindung betrifft eine Produktionslinie zur Dekontamination von fliessbandartig durch eine Dekontaminationseinheit geleiteten Stückgutartikeln mittels einer Dekontaminationsvorrichtung, wobei sich die Dekontaminationseinheit innerhalb eines Containments befindet. Der Dekontaminationseinheit sind eine Zufuhreinheit zum Einbringen der zu dekontaminierenden Stückgutartikel vorgeschaltet und eine Abfuhreinheit zum Weiterleiten dekontaminierter Stückgutartikel nachgeschaltet. Als zu dekontaminierende Stückgutartikel kommen Behältnisse für pharmakologische, biotechnische, chemische oder lebensmittel-technische Substanzen oder Stopfen und Verschlusskappen, insbesondere aus Gummi oder Kunststoff, zum Verschliessen solcher Behältnisse, in Betracht. Die Dekontaminationsvorrichtung kann als Strahlungsquelle oder Begasungseinrichtung ausgebildet sein.

### Stand der Technik

In der Monographie von L. Gail und U. Gommel (Herausgeber): Reinraumtechnik. Springer-Verlag, Berlin, 4. Aufl. 2018, S. 261ff., werden Transfer-Systeme zum Einbringen von vorsterilisierten Stopfen oder Kappen in ein Containment beim automatisierten Abfüllen von Pharmaprodukten beschrieben. Auf S. 275 dieser Monographie ist erwähnt, dass sich für die Oberflächendekontamination von Packmitteln die Behandlung in Elektronenstrahl-Tunneln etabliert hat, insbesondere in der pharmazeutischen Industrie, z.B. für das Einschleusen in aseptisch betriebene Isolatoren. In der WO 2018/109 549 A1 ist dargestellt, wie in einem Containment unter sterilen Bedingungen Verschlüsse für Behältnisse aus einem Stückgut-Separator einem Magazin zugeführt werden, um von dort, auf die in einem parallelen Produktionsschritt mit einer Substanz gefüllten Behältnisse, als verschliessende Stopfen aufzusetzen. Hingegen fehlt es bisher an einer apparativ und produktionstechnisch effizienten und dabei aseptisch zuverlässig arbeitenden Dekontaminationsvorrichtung für die massenweise Behandlung von Stückgutartikeln.

### Aufgabe der Erfindung

Der Erfindung liegt folglich die Aufgabe zugrunde, einen konstruktiven Aufbau einer Produktionslinie zur fliessbandartigen Dekontamination von Stückgutartikeln, wie Behältnisse und deren Verschlusselemente, zu schaffen. Dabei ist vorzusehen, dass sich die Produktionslinie, neben der Erfüllung von Reinheitserfordernissen und wirtschaftlichen Aspekten, bis zur automatisierten Weiterverarbeitung der Behältnisse bzw. Verschlusselemente erstrecken kann.

### Übersicht über die Erfindung

Bei der Produktionslinie zur Dekontamination von fliessbandartig durch eine Dekontaminationseinheit geleiteten Stückgutartikeln mittels einer Dekontaminationsvorrichtung ist die Dekontaminationseinheit innerhalb eines Containments angeordnet. Zum Einbringen der zu dekontaminierenden Stückgutartikel sind der Dekontaminationseinheit eine Zufuhreinheit vorgeschaltet, und zum Weiterleiten dekontaminierter Stückgutartikel eine Abfuhreinheit nachgeschaltet. Die Dekontaminationseinheit weist zur Durchleitung der Stückgutartikel einen ersten Förderer und zumindest einen weiteren Förderer auf, die Andockstellen besitzen, zu denen jeweils ein Pneumatikkanal führt. Der erste Förderer ist angetrieben, um fortlaufend an seinen Andockstellen nächste Stückgutartikel temporär während der beginnenden Dekontamination mittels Unterdruck an einer ersten Artikelfläche zu halten und die bereits erstmals der Wirkung der Dekontaminationsvorrichtung ausgesetzten Stückgutartikel fortlaufend an den zumindest einen weiteren angetriebenen Förderer zu transferieren. Der zumindest eine weitere Förderer hält die vom ersten Förderer zu übernehmenden Stückgutartikel zur Fortsetzung der Dekontamination an einer anderen Artikelfläche, so dass schliesslich die gesamte Oberfläche jedes Stückgutartikels der Wirkung der Dekontaminationsvorrichtung ausgesetzt ist. Der vollständig dekontaminierte Stückgutartikel löst sich von dem zumindest einen weiteren Förderer und gelangt auf ein Transportmittel.

Nachfolgend werden spezielle Ausführungsformen der Erfindung definiert: In der Dekontaminationseinheit mit den beiden vorhandenen Förderern sind weitere derartige Förderer in Reihe verkettet. Der jeweils nächste Förderer ist dazu bestimmt, die zuvor nacheinander an den vorangehenden Förderern gehaltenen und bereits der Wirkung der Dekontaminationsvorrichtung ausgesetzten Stückgutartikel, vom jeweils vorangehenden Förderer zu übernehmen und an jeweils einer anderen Artikelfläche als am vorangehenden Förderer zu halten. Der vollständig dekontaminierte Stückgutartikel löst sich vom letzten Förderer und gelangt auf ein Transportmittel.

Für die Übernahme eines bereits der Wirkung der Dekontaminationsvorrichtung ausgesetzten Stückgutartikels von einem vorangehenden Förderer auf einen nächsten Förderer sind die Pneumatikkanäle des jeweils nächsten Förderers mit jeweils intensiverem Unterdruck beschickt. Alternativ sind Abstreifelemente für das Lösen von bereits an einem vorangehenden Förderer behandelten Stückgutartikel vorgesehen. Oder die Förderer sind mit einer Steuerung verbunden, welche dazu dient, bei Erreichen einer für den bereits behandelten Stückgutartikel zur Übergabe/Übernahme bestimmten Andockstelle den Unterdruck im zugehörigen Pneumatikkanal zu minimieren bzw. abzuschalten.

Benachbarte Förderer sind mit zueinander gegensätzlicher taktweiser oder kontinuierlicher Drehbewegung betrieben.

Die Dekontaminationsvorrichtung ist als eine Strahlungsquelle, z.B. UVC, UV, Elektronen-, Gamma-, Röntgenstrahlung oder Puls-Licht ausgebildet. Eine alternative Dekontaminationsvorrichtung kann als Begasungseinrichtung zur Vernebelung einer Biozid-Lösung -z.B. eine H₂O₂-Lösung - beschaffen sein.

Die Strahlungsquelle ist einerseits der zumindest beiden Förderer bzw. der miteinander verketteten Förderer angeordnet, und andererseits der zumindest beiden Förderer bzw. der miteinander verketteten Förderer sind ein oder mehrere Reflektoren zur Reflektion der von der Strahlungsquelle emittierten Strahlung auf die in den Förderern gehaltenen Stückgutartikeln installiert. Eine andere Konstruktionsvariante ist, mehrere Strahlungsquellen herum um die zumindest beiden Förderer bzw. die miteinander verketteten Förderer anzuordnen, zu denen zusätzlich ein oder mehrere Reflektoren positioniert sein können.

Die Zufuhreinheit umfasst einen Stückgut-Separator mit einer Einfüllöffnung zum Beschicken mit Stückgutartikeln und ein direkt oder indirekt vom Stückgut-Separator zur Dekontaminationseinheit für die separierten Stückgutartikeln leitendes Transportmittel. Die Zufuhreinheit umfasst ferner einen zwischen dem Stückgut-Separator und dem Transportmittel angeordneten Förderer. Das Transportmittel ist bandartig ausgebildet ist, während der Förderer als horizontal oder vertikal aufgestellter Karussellförderer beschaffen ist.

Die Abfuhreinheit weist zumindest ein sich an den zuletzt in der Reihe aufgestellten Förderer anschliessendes bandartiges Transportmittel zur Abfuhr der in der Dekontaminationseinheit dekontaminierten Stückgutartikel auf. Innerhalb der Abfuhreinheit folgt dem sich an den zuletzt in der Reihe aufgestellten Förderer anschliessenden bandartigen Transportmittel zumindest ein weiterer Förderer, in Gestalt eines horizontal oder vertikal aufgestellten Karussellförderers. Hierbei ist der weitere Förderer mit einem nächsten bandartigen Transportmittel verbunden, welches dazu dient, die dekontaminierten Stückgutartikel innerhalb des Containments oder zu einer Prozesskammer in einer Containment-Erweiterung zwecks weiterer Verarbeitung zu transferieren.

Die Art der eingesetzten Dekontaminationsvorrichtung und deren eingestellte Intensität ist an die Art, das Reinheitserfordernis und den beabsichtigten Produktionsumfang der zu dekontaminierenden Stückgutartikel angepasst.

Die zu dekontaminierenden Stückgutartikel betreffen Behältnisse für pharmakologische, biotechnische, chemische oder lebensmittel-technische Substanzen oder Stopfen und Verschlusskappen, insbesondere aus Gummi oder Kunststoff, zum Verschliessen solcher Behältnisse.

Vor einem Produktionsstart ist das Containment bzw. auch die Containment-Erweiterung mit der Dekontaminationseinheit und der Prozesskammer in üblicher Weise dekontaminiert, z.B. mit einer H₂O₂-Begasung. Während der Produktion ist in der Dekontaminationseinheit und der Prozesskammer eine Laminarströmung angelegt.

Während der Produktion sind gegenüber dem äusseren Atmosphärendruck angelegt:
- in der Zufuhreinheit ein kleinerer Überdruck als der in der Dekontaminationseinheit herrschende Überdruck;
- in der Abfuhreinheit ein grösserer Überdruck als der in der Dekontaminationseinheit herrschende Überdruck; und
- in der Prozesskammer der Containment-Erweiterung ein grösserer Überdruck als der in der Abfuhreinheit herrschende Überdruck.

### Kurzbeschreibung der beigefügten Zeichnungen

Es zeigen:

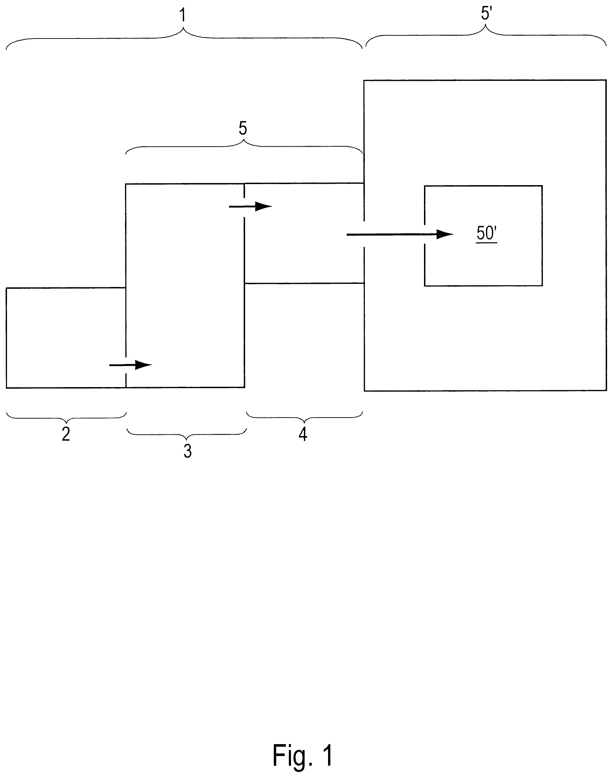

- Figur 1 -: die erfindungsgemässe Produktionslinie zur Dekontamination von fliessbandartig durch eine Dekontaminationseinheit geleiteten Stückgutartikeln, mit Zuführeinheit, damit verbundener Dekontaminationseinheit, sich anschliessender Abfuhreinheit und Übergang zur einer Containment-Erweiterung mit deren Prozesskammer, im schematischen Aufbau;
- Figur 2A -: aus Figur 1 eine *erste Ausführungsform* der Zuführeinheit mit Stückgut-Separator, daran angeschlossenem erstem Förderer und nächstem Transportmittel;
- Figur 2B -: aus Figur 2A der erste Förderer in Vergrösserung mit konstruktiven Details;
- Figur 2C -: eine *zweite Ausführungsform* der Zufuhreinheit mit einem ersten Förderer als Bestandteil des Stückgut-Separators, daran angeschlossenem zweitem Förderer und nächstem Transportmittel;

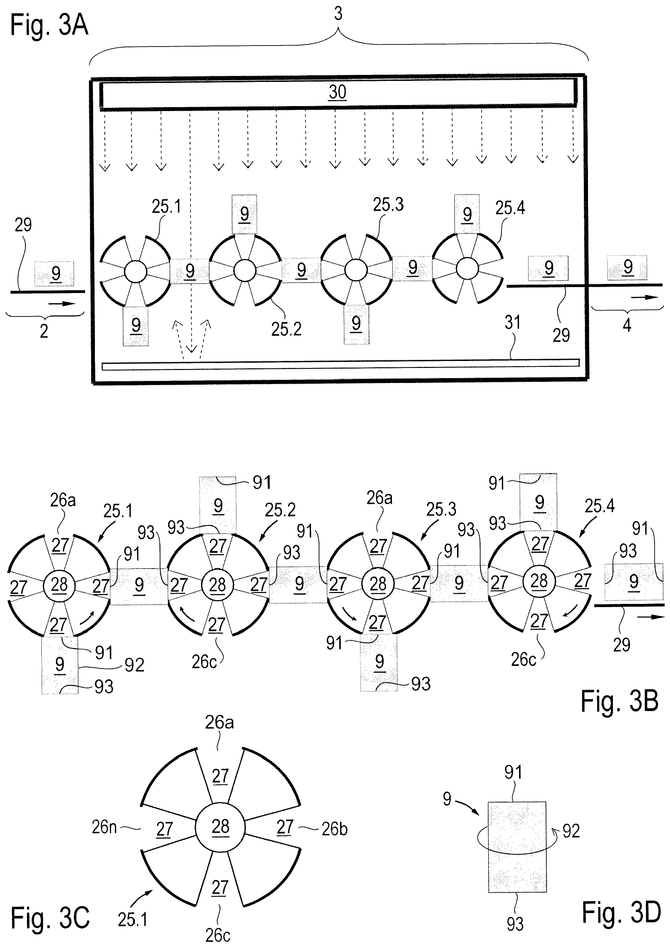

- Figur 3A -: aus Figur 1 den Übergang von der Zuführeinheit zur Dekontaminationseinheit, mit mehreren miteinander verketteten Förderern und Übergang zur Abfuhreinheit;
- Figur 3B -: aus Figur 3A die miteinander verketteten Förderer in Vergrösserung mit konstruktiven Details und Übergang zur Abfuhreinheit;
- Figur 3C -: aus Figur 3A einen der Förderer in weiterer Vergrösserung mit konstruktiven Details;
- Figur 3D -: aus Figur 3A einen Stückgutartikel in Vergrösserung; und
- Figur 4 -: aus Figur 1 die Abfuhreinheit mit zwei miteinander verketteten Förderern und je einem vor- und einem nachgeschalteten Transportmittel.

### Ausführunqsbeispiel

Mit Bezug auf die beiliegenden Zeichnungen erfolgt nachstehend die detaillierte Beschreibung der erfindungsgemässen Produktionslinie zur Dekontamination von fliessbandartig durch eine Dekontaminationseinheit geleiteten Stückgutartikeln. Hierbei können die dekontaminierten Stückgutartikel zur Weiterverarbeitung in der Prozesskammer einer Containment-Erweiterung vorgesehen sein.

### Figur 1

Im prinzipiellen Aufbau betrachtet, beginnt die Produktionslinie **1** zur Dekontamination von fliessbandartig durch eine Dekontaminationseinheit **3** geleiteten Stückgutartikeln **9** mit einer Zuführeinheit **2**, der die damit verbundene Dekontaminationseinheit **3** folgt. Die zu dekontaminierenden Stückgutartikel **9** sind Behältnisse für pharmakologische, biotechnische, chemische oder lebensmittel-technische Substanzen oder Stopfen und Verschlusskappen, insbesondere aus Gummi oder Kunststoff, zum Verschliessen solcher Behältnisse.

An die Dekontaminationseinheit **3** schliesst sich eine Abfuhreinheit **4** an. Die Dekontaminationseinheit **3** zusammen mit der Abfuhreinheit **4** sind in einem Containment **5** untergebracht. Der Ausgang der Abfuhreinheit **4** bildet den Übergang zu einer Containment-Erweiterung **5'** mit deren innerer Prozesskammer **50'**. Die Zuführeinheit **2** und die Abfuhreinheit **4** sind Transportstrecken vor bzw. hinter der Dekontaminationseinheit **3,** in welcher die dekontaminierende Behandlung der Stückgutartikeln **9** mittels Bestrahlung oder Begasung stattfindet. Im hier gezeigten Beispiel gelangen die dekontaminierten Stückgutartikel **9** mittels der Abfuhreinheit **4**, unter Reinraumbedingungen, zu einer Containment-Erweiterung **5'** zwecks weiterer Verarbeitung, z.B. werden Stopfen als sterile Verschlusselemente auf mit pharmakologischer Substanz gefüllte Vials automatisiert aufgesetzt.

Vor einem Produktionsstart wird das Containment **5** bzw. auch die Containment-Erweiterung **5'** mit der Dekontaminationseinheit **3** und der Prozesskammer **50'** in üblicher Weise dekontaminiert, z.B. mit einer H₂O₂-Begasung. Während der Produktion ist in der Dekontaminationseinheit **3** und der Prozesskammer **50'** eine Laminarströmung angelegt. Während der Produktion herrscht gegenüber dem äusseren Atmosphärendruck:
- in der Zufuhreinheit **2** ein kleinerer Überdruck als der in der Dekontaminationseinheit **3** anstehende Überdruck;
- in der Abfuhreinheit **4** ein grösserer Überdruck als der in der Dekontaminationseinheit **3** anstehende Überdruck; und
- in der Prozesskammer **50'** der Containment-Erweiterung **5'** ein grösserer Überdruck als der in der Abfuhreinheit **4** anstehende Überdruck.

### Figuren 2A und 2B

In der einfachsten Version umfasst die Zufuhreinheit 2 einen Stückgut-Separator **20** mit einer Einfüllöffnung **21** zum Beschicken mit Stückgutartikeln **9** und ein direkt vom Stückgut-Separator **20** zur Dekontaminationseinheit **3** leitendes bandartiges Transportmittel **29** zum Einbringen der zu dekontaminierenden Stückgutartikel **9** in die Dekontaminationseinheit **3.**

Diese *erste Ausführungsform* einer erweiterten Zuführeinheit **2** umfasst zunächst einen Stückgut-Separator **20** von schneckenartigem Aufbau mit einer Einfüllöffnung **21** an einem Schacht **22** zum Beschicken mit Stückgutartikeln **9.** Vom Ausgang des Stückgut-Separators **20** werden die Stückgutartikel **9** in geordneter Vereinzelung auf die systematisch verteilten Andockstellen **26a-26n** am Aussenumfang des karussellartigen, sich im Urzeigersinn drehenden Förderers **25.1** transferiert. An jeder Andockstelle **26a-26n** mündet ein Pneumatikkanal **27** mit ansaugender Wirkung. Der jeweils vom Stückgut-Separator **20** freikommende Stückgutartikel **9** wird an der Andockstelle **26y** in deren Hohlform gezogen und darin gehalten. Die Hohlform kann eine verzahnte Kontur, wie dargestellt, oder muldenartig oder speziell der Geometrie der zu verarbeitenden Stückgutartikel **9** angepasst sein.

Eine Steuerung **28** dient dazu, beim Erreichen der Position der Andockstelle **26x** die Sogwirkung abzuschalten, so dass der hier gehaltene Stückgutartikel **9** freigelassen wird, um auf das bandartige Transportmittel **29** zu gelangen, welches die die vereinzelten Stückgutartikel **9** zur Dekontaminationseinheit **3** fördert. Vereinfacht kann alternativ oder zusätzlich an dieser Position ein Abstreifer angeordnet sein, der das Lösen des betreffenden Stückgutartikels **9** aus der Andockstelle **26x** bewirkt.

### Figur 2C

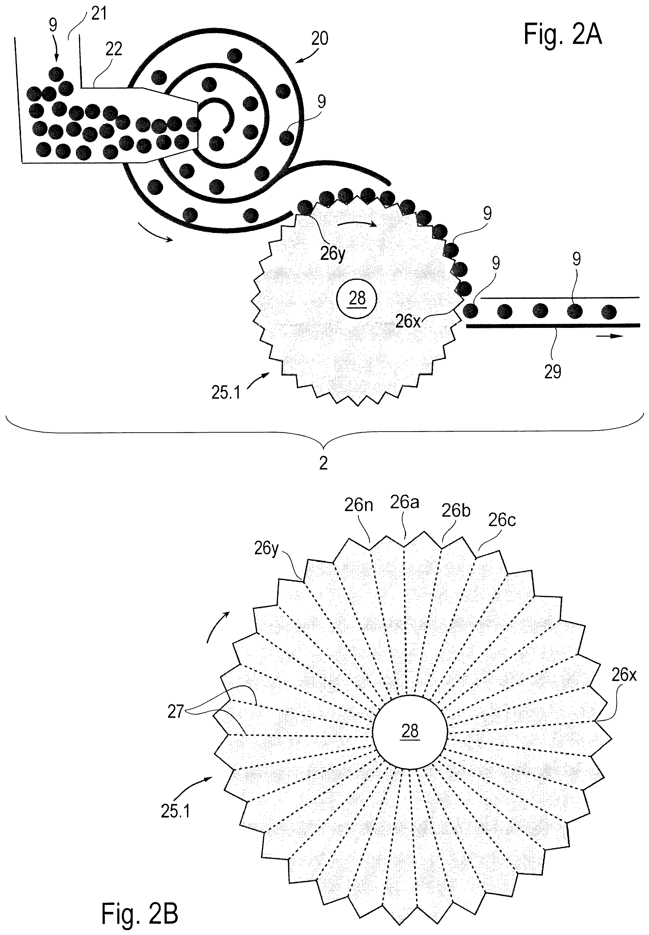

Bei der *zweiten Ausführungsform* einer erweiterten Zuführeinheit **2** ist ein erster Förderer **25.1** Bestandteil des Stückgut-Separators **20**, auf den ein kurvig gestalteter Schacht **22** führt und die Stückgutartikel **9** bereits aufgereiht, in Vereinzelung aufnimmt. Die Beschickung der Zuführeinheit **2** mit Stückgutartikeln **9** erfolgt wiederum über die Einfüllöffnung **21** am Schacht **22.** Vom ersten Förderer **25.1**, welcher im Wesentlichen mit jenem aus Figur 2A identisch ist und sich im Uhrzeigersinn dreht, werden nacheinander Stückgutartikel **9** in der Position der Andockstelle **26y** auf einen zweiten Förderer **25.2** geleitet. Bei diesem sich entgegen dem Uhrzeigersinn drehenden zweiten Förderer **25.2** werden die Andockstellen **26a-26n** nicht von einer am Aussenumfang vorhandenen Sägezahnform, sondern von einem auf der Oberfläche angebrachtem, kreisförmigem Lochraster als Nester für die anzusaugenden Stückgutartikel **9** gebildet. Bezüglich des Transfers der Stückgutartikel **9** vom zweiten Förderer **25.2** auf das sich anschliessende bandartige Transportmittel **29** wird auf die äquivalente Beschreibung zu den Figuren 2A+2B verwiesen.

### Figuren 3A bis 3D

Hier dargestellt sind einerseits der Dekontaminationseinheit **3**, der Übergang von der Zuführeinheit **2** mit dem bandartigen Transportmittel **29**, welches die zu dekontaminierenden Stückgutartikel **9** zur Dekontaminationseinheit **3** letztlich heranführt, und andererseits der Dekontaminationseinheit **3**, der Übergang zur Abfuhreinheit **4** mit dem bandartigen Transportmittel **29**, welches die in der Dekontaminationseinheit **3** behandelten Stückgutartikel **9** aus der Dekontaminationseinheit **3** als erstes abtransportiert. Hauptsächlich dient diese Figurenfolge jedoch dazu, den prinzipiellen inneren apparativen Aufbau und die Funktion der Dekontaminationseinheit **3** beim Dekontaminieren der durchgeschleusten Stückgutartikel **9** - beispielhaft in Gestalt zylindrischer Stopfen mit den Deckflächen **91**, den Mantelflächen **92** und den Bodenflächen **93** - zu erläutern.

Zur Vereinfachung der Zeichnungsfiguren und deren nachstehender Beschreibung weisen die hier gezeigten, vier miteinander verketteten Förderer **25.1-25.4** lediglich jeweils nur vier Andockstellen **26a-26n** und entsprechend vier, an jeweils eine davon, heranführende Pneumatikkanäle **27** auf. In der Realität wird der Fachmann für eine effiziente Produktion eine hohe Anzahl vorsehen und zudem die Pneumatikkanäle **27** wesentlich feiner gestalten.

Der erste Förderer **25.1** dreht sich entgegen dem Uhrzeigersinn, um fortlaufend vom zuführenden Transportmittel **29** an seinen umlaufenden Andockstellen **26a-26n** nächste Stückgutartikel **9** temporär während der beginnenden Dekontamination mittels dem über die Pneumatikkanäle **27** an seinen Mündungen herrschenden Sog an einer ersten Artikelfläche **91**, z.B. der Deckfläche **91**, zu halten. Die bereits erstmals der Wirkung der Dekontaminationsvorrichtung **30** ausgesetzten Stückgutartikel **9** werden fortlaufend an den zweiten, sich im Uhrzeigersinn drehenden Förderer **25.2** transferiert. Hierbei gelangen die Stückgutartikel **9**, quasi gewendet, mit einer anderen Artikelfläche **92**, z.B. der Bodenfläche **92**, vom Sog erfasst, an eine adäquat stehende Andockstelle **26a-26n**.

Bei sich fortsetzender Dekontamination werden in gleicher Weise die insoweit behandelten Stückgutartikel **9** vom zweiten Förderer **25.2** auf den dritten Förderer **25.3** und von diesem auf den vierten Förderer **25.4** weitergereicht. Vorteilhaft werden hierbei die Stückgutartikel **9** am jeweils nächsten Förderer **25.3,25.4** jeweils an einer anderen Artikelfläche **91,93** als am vorangehenden Förderer **25.1-25.3** gehalten. Schliesslich werden die vollständig dekontaminierten Stückgutartikel **9** vom letzten, vierten Förderer **25.4** gelöst und gelangen auf das Transportmittel **29.** Die zueinander benachbarten Förderer **25.1-25.4** werden mit zueinander gegensätzlicher taktweiser oder kontinuierlicher Drehbewegung betrieben.

Für die bereits schrittweise behandelten Stückgutartikel **9** werden für deren Lösen vom vorangehenden Förderer **25.1-25.3** und die Übernahme auf einen nächsten Förderer **25.2-25.4** folgende Alternativen vorgeschlagen:
A1: die Pneumatikkanäle **27** des jeweils nächsten Förderers **25.2-25.4** werden mit jeweils intensiverem Unterdruck beschickt und ziehen somit die Stückgutartikel **9** mit höherer Saugkraft an; oder
A2: zwischen den Förderern **25.1-25.4** bzw. an jedem Förderer **25.1-25.4** ist jeweils ein Abstreifelement vorgesehen; oder
A3: die vorangehenden Förderer **25.1-25.3** sind mit einer Steuerung **28** verbunden, welche bei Erreichen einer für den Stückgutartikel **9** zur Übergabe/Übernahme bestimmten Andockstelle **26b** den Unterdruck im zugehörigen Pneumatikkanal **27** minimiert bzw. abschaltet.

Um die fertig dekontaminierten Stückgutartikel **9** vom letzten Förderer **25.4** zu lösen und auf das Transportmittel **29** zu bringen, kommen ein Abstreifelement gemäss Alternative A2 oder die Steuerung **28** gemäss Alternative A3 in Betracht.

Die Dekontaminationsvorrichtung **30** ist als Strahlungsquelle, z.B. UVC, UV, Elektronen-, Gamma-, Röntgenstrahlung oder Puls-Licht, oder als Begasungseinrichtung zur Vernebelung einer Biozid-Lösung, z.B. eine H₂O₂-Lösung, ausgebildet.

Bei der Verwendung von Strahlungsquellen als Dekontaminationsvorrichtung **30** in der Dekontaminationseinheit **3** werden folgende Alternativen vorgeschlagen:
B1: die Strahlungsquelle ist einerseits der miteinander verketteten Förderer **25.1-25.4** angeordnet, und andererseits der miteinander verketteten Förderer **25.1-25.4** sind ein oder mehrere Reflektoren **31** zur Reflektion der von der Strahlungsquelle emittierten Strahlung auf die in den Förderern **25.1-25.4** gehaltenen Stückgutartikeln **9** installiert; oder
B2: um die miteinander verketteten Förderer **25.1-25.4** herum sind mehrere Strahlungsquellen angeordnet, zu denen zusätzlich ein oder mehrere Reflektoren **31** positioniert sein können.

Die Art der eingesetzten Dekontaminationsvorrichtung **30** und deren eingestellte Intensität wird der Fachmann an die Art, das Reinheitserfordernis und den beabsichtigten Produktionsumfang der zu dekontaminierenden Stückgutartikel **9** anpassen.

### Figur 4

In der einfachsten Version weist die Abfuhreinheit **4** ein sich an den zuletzt in der Reihe aufgestellten Förderer **25.4** anschliessendes bandartiges Transportmittel **29** zur Abfuhr der in der Dekontaminationseinheit **3** dekontaminierten Stückgutartikel **9** auf.

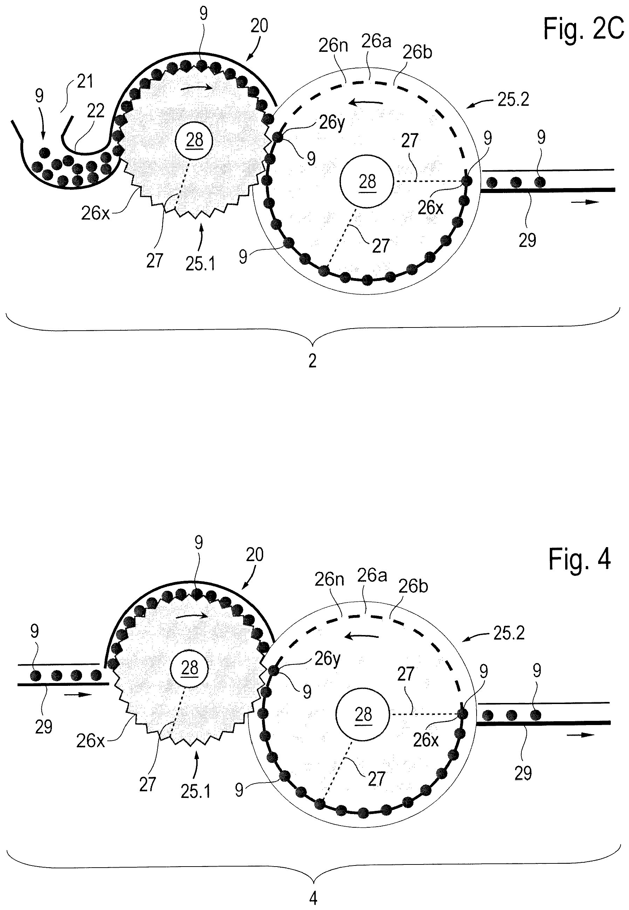

In der erweiterten Version der Abfuhreinheit **4** folgen dem sich an den zuletzt in der Reihe aufgestellten Förderer **25.4** anschliessenden bandartigen Transportmittel **29** zwei in Reihe aufgestellte weitere Förderer **25.1,25.2.** Vom in der Produktionslinie **1** zuvorderst stehenden weiteren Förderer **25.2** übernimmt ein nächstes bandartiges Transportmittel **29** die dekontaminierten Stückgutartikel **9** zum Transfer innerhalb des Containments **5** oder zur Prozesskammer **50'** in einer Containment-Erweiterung **5'** zwecks weiterer Verarbeitung. Hinsichtlich der Konstruktion und dem Zusammenwirken der beiden weiteren Förderer **25.1,25.2** innerhalb der Abfuhreinheit **4** wird auf die vorherige Beschreibung zur Figur 2C verwiesen.

## Patentansprüche

1. Produktionslinie (**1**) zur Dekontamination von fliessbandartig durch eine Dekontaminationseinheit (**3**) geleiteten Stückgutartikeln (**9**) mittels einer Dekontaminationsvorrichtung (**30**), wobei:
a) die Dekontaminationseinheit (**3**) sich innerhalb eines Containments (**5**) befindet;
b) der Dekontaminationseinheit (**3**) eine Zufuhreinheit (**2**) zum Einbringen der zu dekontaminierenden Stückgutartikel (**9**) vorgeschaltet ist;
c) der Dekontaminationseinheit (**3**) eine Abfuhreinheit (**4**) zum Weiterleiten dekontaminierter Stückgutartikel (**9**) nachgeschaltet ist, **dadurch gekennzeichnet, dass**
d) die Dekontaminationseinheit (**3**) zur Durchleitung der Stückgutartikel (**9**) einen ersten Förderer (**25.1**) und zumindest einen weiteren Förderer (**25.2**) aufweist, die Andockstellen (**26a-26n**) besitzen, zu denen jeweils ein Pneumatickanal (**27**) führt;
e) der erste Förderer (**25.1**) angetrieben ist, um fortlaufend an seinen Andockstellen (**26a-26n**) nächste Stückgutartikel (**9**) temporär während der beginnenden Dekontamination mittels Unterdruck an einer ersten Artikelfläche (**91**) zu halten und die bereits erstmals der Wirkung der Dekontaminationsvorrichtung (**30**) ausgesetzten Stückgutartikel (**9**) fortlaufend an den zumindest einen weiteren angetriebenen Förderer (**25.2**) zu transferieren; wobei
f) der zumindest eine weitere Förderer (**25.2**) die vom ersten Förderer (**25.1**) zu übernehmenden Stückgutartikel (**9**) zur Fortsetzung der Dekontamination an einer anderen Artikelfläche (**92,93**) hält, so dass schliesslich die gesamte Oberfläche jedes Stückgutartikels (**9**) der Wirkung der Dekontaminationsvorrichtung (**30**) ausgesetzt ist; und
g) der vollständig dekontaminierte Stückgutartikel (**9**) sich von dem zumindest einen weiteren Förderer (**25.2**) löst und auf ein Transportmittel (**29**) gelangt.

2. Produktionslinie (**1**) nach Anspruch 1, **dadurch gekennzeichnet, dass** in der Dekontaminationseinheit (**3**) mit den beiden vorhandenen Förderern (**25.1+25.2**) weitere derartige Förderer (**25.3+25.4**) in Reihe verkettet sind, wobei:
a) der jeweils nächste Förderer (**25.2-25.4**) dazu bestimmt ist, die zuvor nacheinander an den vorangehenden Förderern (**25.1-25.3**) gehaltenen und bereits der Wirkung der Dekontaminationsvorrichtung (**30**) ausgesetzten Stückgutartikel (**9**), vom jeweils vorangehenden Förderer (**25.1-25.3**) zu übernehmen und an jeweils einer anderen Artikelfläche (**91-93**) als am vorangehenden Förderer (**25.1-25.3**) zu halten; und
b) der vollständig dekontaminierte Stückgutartikel (**9**) sich vom letzten Förderer **(25.4)** löst und auf ein Transportmittel (**29**) gelangt.

3. Produktionslinie (**1**) nach Anspruch 2, **dadurch gekennzeichnet, dass** für die Übernahme eines bereits der Wirkung der Dekontaminationsvorrichtung (**30**) ausgesetzten Stückgutartikels (**9**) von einem vorangehenden Förderer (**25.1-25.3**) auf einen nächsten Förderer (**25.2-25.4**):
a) die Pneumatikkanäle (**27**) des jeweils nächsten Förderers (**25.2-25.4**) mit jeweils intensiverem Unterdruck beschickt sind; oder
b) Abstreifelemente für das Lösen von bereits an einem vorangehenden Förderer (**25.1-25.3**) behandelten Stückgutartikel (**9**) vorgesehen sind; oder
c) die Förderer (**25.1-25.3**) mit einer Steuerung (**28**) verbunden sind, welche dazu dient, bei Erreichen einer für den bereits behandelten Stückgutartikel (**9**) zur Übergabe/Übernahme bestimmten Andockstelle (**26b**) den Unterdruck im zugehörigen Pneumatikkanal (**27**) zu minimieren bzw. abzuschalten.

4. Produktionslinie (**1**) nach zumindest einem der Ansprüche 1 bis 3, **dadurch gekennzeichnet, dass** benachbarte Förderer (**25.1-25.4**) mit zueinander gegensätzlicher taktweiser oder kontinuierlicher Drehbewegung betrieben sind.

5. Produktionslinie (**1**) nach zumindest einem der Ansprüche 1 bis 4, **dadurch gekennzeichnet, dass** die Dekontaminationsvorrichtung (**30**) ausgebildet ist als:
a) eine Strahlungsquelle, z.B. UVC, UV, Elektronen-, Gamma-, Röntgenstrahlung oder Puls-Licht; oder
b) eine Begasungseinrichtung zur Vernebelung einer Biozid-Lösung, z.B. eine H₂O₂-Lösung.

6. Produktionslinie (**1**) nach Anspruch 5, **dadurch gekennzeichnet, dass**
a) die Strahlungsquelle einerseits der zumindest beiden Förderer (**25.1+25.2**) bzw. der miteinander verketteten Förderer (**25.1-25.4**) angeordnet ist, und andererseits der zumindest beiden Förderer (**25.1+25.2**) bzw. der miteinander verketteten Förderer (**25.1-25.4**) ein oder mehrere Reflektoren (**31**) zur Reflektion der von der Strahlungsquelle emittierten Strahlung auf die in den Förderern (**25.1+25.2;25.1-25.4**) gehaltenen Stückgutartikeln (**9**) installiert sind; oder
b) mehrere Strahlungsquellen herum um die zumindest beiden Förderer (**25.1+25.2**) bzw. die miteinander verketteten Förderer (**25.1-25.4**) angeordnet sind, zu denen zusätzlich ein oder mehrere Reflektoren (**31**) positioniert sein können.

7. Produktionslinie (**1**) nach zumindest einem der Ansprüche 1 bis 6, **dadurch gekennzeichnet, dass** die Zufuhreinheit (**2**) umfasst:
a) einen Stückgut-Separator (**20**) mit einer Einfüllöffnung (**21**) zum Beschicken mit Stückgutartikeln (**9**); und
b) ein direkt oder indirekt vom Stückgut-Separator (**20**) zur Dekontaminationseinheit (**3**) für die separierten Stückgutartikeln (**9**) leitendes Transportmittel (**29**).

8. Produktionslinie (**1**) nach Anspruch 7, **dadurch gekennzeichnet, dass** die Zufuhreinheit (**2**) ferner einen zwischen dem Stückgut-Separator (**20**) und dem Transportmittel (**29**) angeordneten Förderer (**25.1**) umfasst.

9. Produktionslinie (**1**) nach zumindest einem der Ansprüche 2, 7 und 8, **dadurch gekennzeichnet, dass** das Transportmittel (**29**) bandartig ausgebildet ist, während der Förderer (**25.1**) als horizontal oder vertikal aufgestellter Karussellförderer beschaffen ist.

10. Produktionslinie (**1**) nach zumindest einem der Ansprüche 1 bis 9, **dadurch gekennzeichnet, dass** die Abfuhreinheit (**4**) zumindest ein sich an den zuletzt in der Reihe aufgestellten Förderer (**25.2;25.4**) anschliessendes bandartiges Transportmittel (**29**) zur Abfuhr der in der Dekontaminationseinheit (**3**) dekontaminierten Stückgutartikel (**9**) aufweist.

11. Produktionslinie (**1**) nach Anspruch 10, **dadurch gekennzeichnet, dass**
a) innerhalb der Abfuhreinheit (**4**) dem sich an den zuletzt in der Reihe aufgestellten Förderer (**25.2;25.4**) anschliessenden bandartigen Transportmittel (**29**) zumindest ein weiterer Förderer (**25.1,25.2**), in Gestalt eines horizontal oder vertikal aufgestellten Karussellförderers, folgt; wobei
b) der weitere Förderer (**25.1,25.2**) mit einem nächsten bandartigen Transportmittel (**29**) verbunden ist, welches dazu dient, die dekontaminierten Stückgutartikel (**9**) innerhalb des Containments (**5**) oder zu einer Prozesskammer (**50'**) in einer Containment-Erweiterung (**5'**) zwecks weiterer Verarbeitung zu transferieren.

12. Produktionslinie (**1**) nach zumindest einem der Ansprüche 1 bis 11, **dadurch gekennzeichnet, dass** die Art der eingesetzten Dekontaminationsvorrichtung (**30**) und deren eingestellte Intensität an die Art, das Reinheitserfordernis und den beabsichtigten Produktionsumfang der zu dekontaminierenden Stückgutartikel (**9**) angepasst ist.

13. Produktionslinie (**1**) nach zumindest einem der Ansprüche 1 bis 12, **dadurch gekennzeichnet, dass** die zu dekontaminierenden Stückgutartikel (**9**) Behältnisse für pharmakologische, biotechnische, chemische oder lebensmittel-technische Substanzen oder Stopfen oder Verschlusskappen, insbesondere aus Gummi oder Kunststoff, zum Verschliessen solcher Behältnisse sind.

14. Produktionslinie (**1**) nach zumindest einem der Ansprüche 1 bis 13, **dadurch gekennzeichnet, dass**
a) vor einem Produktionsstart das Containment (**5**) bzw. auch die Containment-Erweiterung (**5'**) mit der Dekontaminationseinheit (**3**) und der Prozesskammer (**50'**) in üblicher Weise dekontaminiert ist, z.B. mit einer H₂O₂-Begasung; und
b) während der Produktion in der Dekontaminationseinheit (**3**) und der Prozesskammer (50') eine Laminarströmung angelegt ist.

15. Produktionslinie (1) nach zumindest einem der Ansprüche 1 bis 14, **dadurch gekennzeichnet, dass** während der Produktion gegenüber dem äusseren Atmosphärendruck:
a) in der Zufuhreinheit (2) ein kleinerer Überdruck angelegt ist, als der in der Dekontaminationseinheit (3) herrschende Überdruck;
b) in der Abfuhreinheit (4) ein grösserer Überdruck angelegt ist, als der in der Dekontaminationseinheit (**3**) herrschende Überdruck; und
c) in der Prozesskammer (**50'**) der Containment-Erweiterung (**5'**) ein grösserer Überdruck angelegt ist, als der in der Abfuhreinheit (**4**) herrschende Überdruck.
